(19) Europäisches Patentamt
European Patent Office
Office européen des brevets

(11) Numéro de publication : **0 281 435 B1**

(12) # FASCICULE DE BREVET EUROPEEN

(45) Date de publication du fascicule du brevet : **01.04.92 Bulletin 92/14**

(51) Int. Cl.$^5$ : **C07F 7/08,** A61K 31/695

(21) Numéro de dépôt : **88400170.2**

(22) Date de dépôt : **27.01.88**

(54) **Produit thérapeutique à base de dérivés organiques du silicium.**

(30) Priorité : **06.02.87 FR 8701446**

(43) Date de publication de la demande : **07.09.88 Bulletin 88/36**

(45) Mention de la délivrance du brevet : **01.04.92 Bulletin 92/14**

(84) Etats contractants désignés : **DE GB NL**

(56) Documents cités :
**EP-A- 0 180 505**
**FR-A- 1 234 213**
**FR-M- 6 871**

(73) Titulaire : **Gueyne, Jean**
**Périgord 1 6 Lacets Saint Léon**
**Monte-Carlo (MC)**
Titulaire : **Seguin, Marie-Christine**
**Périgord 1 6 Lacets Saint-Léon**
**Monte-Carlo (MC)**
Titulaire : **Henrotte, Jean Georges**
**11 rue de la Ferronnerie**
**F-75001 Paris (FR)**

(72) Inventeur : **Gueyne, Jean**
**Périgord 1 6 Lacets Saint Léon**
**Monte-Carlo (MC)**
Inventeur : **Seguin, Marie-Christine**
**Périgord 1 6 Lacets Saint-Léon**
**Monte-Carlo (MC)**
Inventeur : **Henrotte, Jean Georges**
**11 rue de la Ferronnerie**
**F-75001 Paris (FR)**

(74) Mandataire : **Kohn, Armand c/o S.A. FEDIT-LORIOT ET AUTRES et al CONSEILS EN PROPRIETE INDUSTRIELLE 38, avenue Hoche**
**F-75008 Paris (FR)**

**EP 0 281 435 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un nouveau produit à usage therapeutique, renfermant comme agent actif un ou plusieurs composés organo-siliciés, et plus particulièrement des silanols. Le produit suivant l'invention convient notamment à la préparation de médicaments agissant comme activateurs et régulateurs du métabolisme, de la croissance et de la multiplication des cellules, en particulier celles qui sont impliquées dans les processus immunitaires ou dans la formation du tissu conjonctif et du tissu osseux.

Plusieurs esters et complexes de silanols, présentant des propriétés thérapeutiques particulières, fort intéressantes, ont éte décrits et utilisés dans le passé ; tel est, par exemple, le cas du salicylate de monométhylsilanetriol et des dérivés du glycérol de polyalcoxysilanes.

Dans le brevet FR-M-6871 on a décrit des solutions aqueuses de composés de silanols avec différents acides, minéraux et organiques, parmi lesquels figurent l'acide glutamique, l'hydroxy-5 tryptophane et la cystéine ; ces solutions ne sont stables qu'à des pH acides, surtout de 4 à 5. La préparation de telles solutions est décrite dans le FR 1 234 213 et comporte un traitement avec un échangeur d'ions, le milieu étant toujours acide, de préférence à pH 3 à 5. Aucun de ces brevets ne montre une action sur la multiplication de cellules ou l'activation du système immunitaire au moyen des solutions concernées. Des cosmétiques sont décrits dans le EP 0 180 505, comprenant une solution aqueuse à mélanger avec une solution grasse, sur la peau ; la phase aqueuse contient du manuronate de silanol, donc du silanol déjà combiné avec un polysaccharide, et des amino-acides végétaux dont la nature n'est pas précisée ; il n'y est pas question de complexe d'un silanol avec un amino-monoacide, en solution aqueuse, ni d'une des activités susmentionnées.

La présente invention met en évidence des applications imprévues et des propriétés thérapeutiques nouvelles, mentionnées plus haut, de produits nouveaux plus actifs que ceux qui ont été décrits précédemment.

L'invention résulte de la constatation que l'activité biologique des atomes de silicium, combinés sous une forme organique, peut être modifiée et orientée par la nature des molécules liées à ces atomes. Ainsi, l'action d'un complexe organique d'un silanol est-elle d'autant plus physiologique, c'est-à-dire d'autant plus proche de l'action naturelle du Si dans la cellule vivante, que le silanol est lié à une molécule naturelle.

Le nouveau produit thérapeutique suivant l'invention, qui comprend un dérivé organique du silicium et un amino-acide, est caractérisé en ce qu'il est constitué par une combinaison d'un silanol avec un amino monoacide, sans fonction phénol, nithiol, libre ou à l'état d'un sel pharmaceutiquement acceptable, ou sous la forme d'un protide.

On a proposé, dans le passé, des complexes thérapeutiques de silanols avec différents acides, mais - comme il ressort du brevet français médical 6 871 M - seuls des aminodiacides ou des acides aminés à fonction phénol ou thiol étaient envisagés. De même, dans F. 1 234 213 seulement un diacide aminé, l'acide glutamique, est considéré comme réactif utile à combiner avec des silanols. La présente invention, par contre, repose sur la constatation que des combinaisons silanol-monoacide aminé, exempt de fonction phénol ou thiol, présentent des avantages thérapeutiques qu'illustrent les exemples 7 à 12 donnés plus loin.

Ainsi, le produit suivant l'invention peut-il être constitué par un complexe ou un composé défini, résultant de la combinaison d'un silanol avec un aminoacide ou avec un protide. L'acide aminé ou le protide peut - bien entendu - porter des substituants ; cela présente, en particulier, de l'utilité dans les cas des acides ou protides insolubles ou peu solubles dans l'eau ; le blocage de leur fonction acide ou amine peut permettre l'obtention de composés solubles.

Des aminoacides, convenant à la réalisation de l'invention, sont par exemple : sérine, thréonine, homosérine, proline, lysine, hydroxylysine, histidine, glycocolle, glutamine, asparagine, arginine, ornithine, ou autres mono-acides aminés non phénoliques et sans groupe Thiol.

Parmi les aminoacides optiquement actifs, les molécules lévogyres sont préférées. Les produits formés par des complexes de silanols avec le l-sérine, l-thréonine, l-hydroxylysine, l-lysine, par exemple, sont particulièrement intéressants.

Les complexes suivant l'invention, dans lesquels l'aminoacide se trouve sous une forme combinée,sont ceux des organo-siliciés avec des protides, -notamment protéines, peptides et polypeptides, - dont un ou plusieurs groupes réactifs, en particulier -COOH, $>$CHOH et $-NH_2$ sont complexés ou combinés avec le composé du silicium. Tels,sont, par exemple,les cas de la disérine et de la caséine. L'invention couvre également les complexes des organosiliciés avec les protides, lorsque ceux-ci sont glycosylés, comme c'est souvent le cas des protides naturels (glycoprotéines, glycopeptides). Tel est par exemple le cas du N-acétylmuramyl-l-alanyl-d-isoglutamine encore appelé muramyl-dipeptide.

Les produits suivant l'invention peuvent être obtenus par la mise en contact, en solution ou en dispersion, d'un ou de plusieurs organo-siliciés particulièrement silanols, avec un ou plusieurs acides aminés, protides ou glycoprotides, définis plus haut.

Les proportions molaires du composé organo-silicié et de l'acide aminé, dans le complexe suivant l'invention, peuvent varier assez largement ; on peut avoir, notamment 0,25 à 2 atomes de Si par groupe -COOH, $>$ CHOH ou-$NH_2$ de l'amino-acide ou protide du complexe ;cependant, les rapports les plus favorables se situent aux environs de 0,25 à 0,50 atomes de Si par groupe -COOH, $>$ CHOH ou -$NH_2$. La concentration de la solution en Si est en général de 0,1 à 1,4 g/l.
Les organo-siliciés et leurs dérivés métalliques peuvent répondre à la formule : $R_xSi(OM)_{(4-x)}$ où R est un alkyle, alcényle ou aryle, X est un nombre de 0 à 3, M désignant un atome de métal alcalin ou d'hydrogène, ou bien un groupe organique à fonction alcool, acide, amine. Lorsque R est un alkyle ou alcényle, il comporte de préférence 1 à 18 atomes de carbone ou - mieux - 1 à 4C. Si R est un aryle il est en $C_6$ ou $C_{10}$, sans compter les substituants alkyliques en $C_1$ à $C_6$ qu'il peut porter.

Des polysilanols, comme ceux dont il est question dans la publication FR2230376, soit

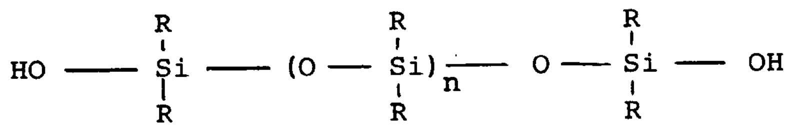

(n = 0 à 20) peuvent être employés ; il est cependant préférable de se servir alors de tels polysilanols ayant au maximum 4 atomes de Si.

Les organo-siloxanes, pouvant être utilisés dans les compositions suivant l'invention, font l'objet du FR 1179743. Dans leur formule générale de polymère

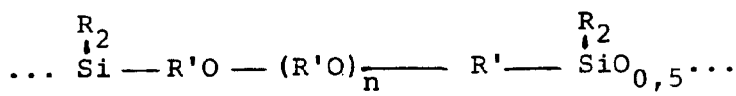

R répond à la définition donnée plus haut, R′ est un alkylène en $C_1$ à $C_4$, n a une valeur de 0 à 20.

Des siloxanes monomères peuvent avoir la structure indiquée dans la publication FR2484425 pages 2 à 4, soit une formule générale

$$Z — RO — (R^1O)_n — R^2 — Y$$

où au moins un des groupes Z et Y est siloxanique , l'autre pouvant être un H. Z ou/et Y peuvent ainsi être

$R_3$ — Si — O 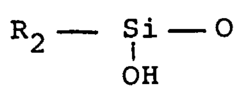 ou 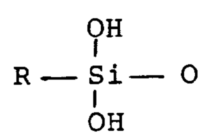

et le composé peut, par exemple, présenter la forme :

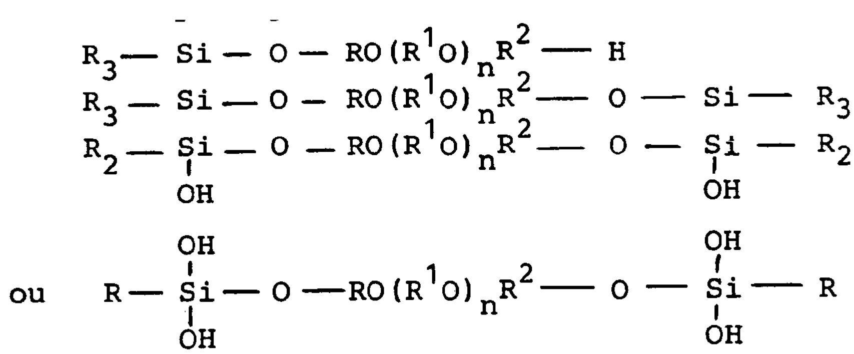

n étant 0 à 20 et le plus souvent 1 à 6.

EXEMPLE 1

Préparation d'un complexe silanol/l-thréonine

On prépare 1 litre de solution aqueuse du complexe, en mélangeant 500 ml renfermant 5,6 g de thréonine avec 500 ml de solution de 4,4g de méthylsilanetriol ; la solution renferme 1,3 g Si par litre, soit 1 mol. de $CH_3Si(OH)_3$ pour 1 mol. de

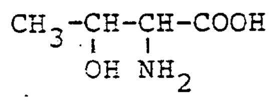

EXEMPLE 2

Préparation d'un complexe silanol/l-sérine

A une solution de 5,3 g, soit 0,0504 mole, de l-sérine

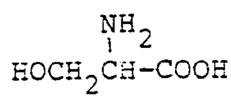

dans 250 ml d'eau à 25°C on ajoute 750 ml d'une solution aqueuse de 4,7 g, soit 0,0499 mole de méthylsilanetriol $CH_3Si~(OH)_3$ à 25°C. On obtient ainsi 1 litre de solution à 10 g/l (c'est-à-dire 1% en poids/volume) de complexe équimolaire

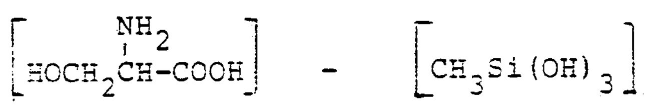

à 1 atome Si par groupe -COOH ou $>$CHOH soit 1,4 Si par litre de solution.

EXEMPLE 3

Préparation d'un complexe silanol-disérine

Une solution aqueuse à 10g/l est préparée à partir de 6,8 g de disérine avec 3,2 g de diméthylsilanediol. Elle tire 0,95 g Si/l.

EXEMPLE 4

Préparation d'un complexe silanol-muramyl-dipeptide

Selon le mode opératoire des exemples précédents, on prépare une solution aqueuse du complexe à partir de 335 mg de méthyl silanetriol par litre (soit 100 mg/l de Si) et de 360 mg de muramyl-dipeptide par litre. Compte tenu des poids moléculaires du Si et du muramyl-dipeptide (P.M. $\simeq$ 500), ce complexe comporte 5 atomes de Si par molécule de muramyl-dipeptide.

EXEMPLE 5

Préparation d'un complexe silanol-caséine

A une solution à pH 11 de 6,5 g de caséine dans 1 litre d'eau on ajoute 5,4 g d'éthylsilane-triol. Le pH du mélange est ensuite ramené à 7 au moyen d'HCl. On obtient ainsi une solution de complexe silanol-caséine

à 1,4 g Si/litre.

EXAMPLE 6

Préparation d'un complexe silanol-hydroxyproline

Suivant le mode opératoire des exemples précédents, on a préparé une solution aqueuse à 10 g/l de complexe

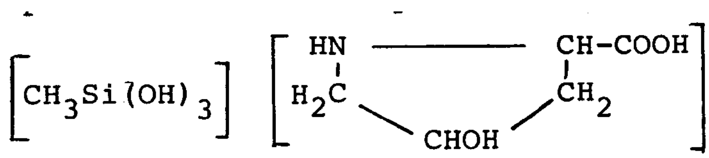

à partir de 5,8 g de l.hydroxyproline et 4,2 g de méthylsilanetriol. La solution titre 1,25 g Si/litre.

EXEMPLE 7

Essais du complexe silanol-thréonine en vue de son application à la multiplication cellulaire

Les essais portent sur des cultures de lymphocytes humains, circulants, provenant de 25 sujets sains, âgés de 21 à 42 ans. Les lymphocytes sont isolés selon la technique classique de Boyum et mis en suspension dans le milieu habituel, RPMI 1640, additionné de 0,5% de mélange pénicilline-streptomycine et de 10% de sérum de veau foetal. Chacune de ces 25 cultures est divisée en 4 fractions A, B, C et D.

(A) La fraction A est subdivisée en 3 sous-fractions $A_1$,$A_2$, $A_3$ auxquelles on ajoute de la solution du complexe selon l'exemple 1, de façon à avoir, par litre de culture :

A1 ............. 1 mg de Si
A2 ............. 5 mg de Si
A3 .............10 mg de Si

Chacune de ces sous-fractions constitue une microculture, dont le volume final est de 220 microlitres, et qui est incubée pendant 72 heures à 37°C en atmosphère saturée à 5% de $CO_2$.

(B) On opère également sur trois sous-fractions $B_1$,$B_2$ et $B_3$ auxquelles on ajoute soit une solution de silanol seul (sans thréonine), soit du silanol complexé avec du salicylate de Na. Comme précédemment, on ajoute des quantités variables de solution de façon à avoir par litre de culture :

$B_1$ ............. 1 mg Si/litre
$B_2$ ............. 5 mg Si/litre
$B_3$ .............10 mg Si/litre

On procède ensuite comme en (A).

(C) A la fraction C de la culture initiale de lymphocytes, on ajoute de la thréonine seule (sans silanol) à la place du complexe et l'on procède comme en (A).

(D) La quatrième fraction D de la culture initiale n'est additionnée ni de complexe silanol-thréonine,ni de silanol seul ou silanol salicylate ni de thréonine. Elle est incubée à 37°C pendant 72 heures comme en (A).

Chaque essai est fait en triple exemplaire.

Dans tous les cas (A,B,C et D),6 heures avant la fin de l'incubation, 0,5 microcuries de thymidine tritiée sont ajoutées aux 220 microlitres de chaque micro-culture. A la fin des 72 heures d'incubation, les micro- cultures sont récoltées et le comptage de la radioactivité émise par la thymidine tritiée est mesuré avec un compteur à scintillation. Le nombre de coups par minute de chacune des microcultures faites en (A), (B) et (C) est rapporté au nombre de coups par minute obtenus par la culture témoin (D). Ce rapport est appelé indice de stimulation

(I.S.) et permet d'estimer l'effet des produits étudiés sur la prolifération cellulaire, puisque l'incorporation de la thymidine dans les cellules est proportionnelle à leur prolifération. L'effet sur la prolifération est nul à I.S.=1, l'effet est stimulateur si I.S. > 1 et l'effet est inhibiteur si I.S. < 1.

Dans les conditions décrites, l'indice de stimulation ne varie pas significativement selon la concentration en Si (pas de différences significatives entre $A_1$, $A_2$, $A_3$, etc.). C'est donc la valeur moyenne des trois sous-fractions qui a été utilisée pour les calculs suivants. Dans ces conditions :

(A) L'indice de stimulation est supérieur ou égal à 3 (c'est-à-dire la multiplication cellulaire est supérieure ou égale à 3 fois celle des cultures témoins D) dans 68% des 25 cultures additionnés de silanol-thréonine selon l'exemple 1.

(B) L'indice de stimulation est supérieur ou égal à 3, dans respectivement 36 et 20% des 25 cultures faites en présence de silanol seul et de silanol-salicylate.

(C) L'indice de stimulation est toujours voisin de 1.

Il résulte de ces observations que les solutions de complexe selon l'exemple 1 stimulent considérablement la multiplication des lymphocytes. Cette stimulation est beaucoup plus grande que la stimulation obtenue sous l'effet du silanol seul ou du silanol-salicylate. La thréonine seule n'a aucun effet sur des lymphocytes en culture puisqu'aucune modification n'est constatée par rapport à l'essai témoin D (I.S. $\simeq$ 1).

La solution de complexe selon l'exemple 1 est utilisable dans des ampoules scellées à une concentration de 1 à 10 mg de Si par litre, destinées à l'administration parentérale.

EXEMPLE 8

Essais du complexe silanol-sérine en vue de ses applications sur la multiplication cellulaire

La solution, préparée suivant l'exemple 2, est diluée avec de l'eau stérile et ajoutée à des cultures de cellules, de façon à titrer 10 mg de Si par litre de culture. Son action a été étudiée sur des cultures de cellules lymphoblastoïdes humaines LDV/7 selon une technique semblable à celle de l'exemple 7.

A court terme, c'est-à-dire au bout de 24 et 48 heures, le complexe diminue considérablement l'incorporation de thymidine tritiée dans les cultures de cellules lymphoblastoïdes (-68% à 24 heures et -67% à 48 heures).

Le silanol seul a une action similaire mais moins intense (-18,5% à 48 heures). Par contre la sérine seule favorise la multiplication des cellules lymphoblastoïdes (+45% à 24 heures et +72% à 48 heures). Le complexe silanol-sérine, et à un moindre titre le silanol seul, s'opposent donc à une prolifération anarchique de cellules de nature cancéreuse, alors que la sérine seule a un effet contraire. A long terme, c'est-à-dire au bout d'une semaine ou deux, aucun effet sur la croissance des cultures de cellules lymphoblastoïdes humaines n'a été noté. Par contre, il y a formation d'agrégats cellulaires. Cela suggère que le complexe se comporte comme un agent de liaison intercellulaire ou tout au moins favorise la formation de jonctions intercellulaires. La sérine seule, le silanol seul et le complexe silanol-salicylate n'ont dans les mêmes conditions opératoires aucun effet sur l'agrégation cellulaire. Le complexe normalise donc les cultures de cellules tumorales, puisque celles-ci sont caractérisées par l'absence de jonctions intercellulaires, dont la présence est notoire dans les cultures de cellules saines.

Notons également que l'absence d'action à long terme du complexe sur la croissance des cellules lymphoblastoïdes peutêtre attribué au fait que l'addition de ce complexe au milieu de culture n'est faite qu'une seule fois au temps O. Le complexe est donc ensuite probablement métabolisé par les cellules et se trouve, après 1 ou 2 semaines de culture, sous une forme différente de la forme initiale. La solution aqueuse initiale du complexe selon l'exemple 2, titrant 10 mg Si par litre, se présente, en particulier, sous la forme d'ampoules scellées de 10 ml. Elle peut recevoir des applications thérapeutiques tant chez l'homme que chez l'animal.

EXEMPLE 9

Essais du complexe silanol-disérine en vue de ses applications en tant qu'agent activateur de la croissance cellulaire.

La solution préparée selon l'exemple 3, titrant 0,95 g Si/litre, est diluée et ajoutée à des cultures de fibroblastes humains de façon à titrer 10 mg de Si/litre de culture, selon une technique semblable à celle de l'exemple 7. Après 72 heures de culture, le complexe augmente nettement l'incorporation de thymidine tritiée (+ 122%). L'effet du silanol seul est moindre (+72%) et celui de la disérine est négligeable (+15%).

Les complexes, selon les exemples 1, 2 et 3, ont donc des propriétés régulatrices sur la croissance et la prolifération cellulaire : ils stimulent la prolifération des cellules quiescentes (lymphocytes circulants) mais ils

inhibent la multiplication de cellules de nature cancéreuse en croissance rapide et continue (cellules lymphoblastoïdes). Ils normalisent aussi la croissance de ces cellules cancéreuses en favorisant leur agrégation. Le silanol seul a des effets similaires mais moins importants.

EXEMPLE 10

Essais des complexes silanol-sérine et silanol-muramyl dipeptide en vue deleurs applications en tant qu'agents activateurs des macrophages et des cellules du système réticulo-endothélial

L'infection brucellique expérimentale permet de tester l'activité des cellules du système réticulo-endothélial (macrophages et autres cellules douées d'activité phagocytaire) et leur capacité de destruction de bactéries pathogènes intracellulaires, telles que Brucella. Des souris femelles CD1 pesant de 20 à 22 g sont utilisées. Au jour 0, tous les animaux sont infectés par l'injection intrapéritonéale de 0,3 ml d'une suspension contenant au total 30 000 bactéries virulantes de Brucella abortus 544. Après 20 jours, les souris sont groupées en 6 lots de 10 animaux.

(A) - Les animaux du lot A subissent alors une injection intrapéritonéale du complexe préparé selon l'exemple 2 (silanol-sérine) de façon à recevoir un total de 0,03 mg Si.

(B)-(C). De même, les animaux des lots B et C reçoivent une quantité identique de Si administrée respectivement sous forme du complexe préparé selon l'exemple 5 (silanol-muramyl-dipeptide), et sous celle de silanol seul.

(D) - Les animaux du lot D reçoivent de la sérine seule.

(E) - Ceux du lot E du muramyl-dipeptide seul et ceux

(F) - du lot F, constituant le lot témoin, reçoivent du salin. Le même traitement est répété tous les jours pendant 15 jours, c'est-à-dire du 20ième au 34ième jour de l'expérience. Au 35ième jour,les animaux sont sacrifiés ; les rates sont prélevées aseptiquement et broyées individuellement. Des ensemencements sur agar-agar sont réalisés à partir des broyats obtenus et placés à 37°C pendant 5 jours. Les résultats sont exprimés en nombre de colonies de Brucella, obtenus par centième de rate. Le nombre de colonies obtenues à partir des rates des 10 animaux du lot témoin F est égal en moyenne à 277. Les résultats obtenus à partir des autres lots de souris sont exprimés en pourcentages du lot témoin F :

| | % |
|---|---|
| A-silanol/sérine ............. | 46 |
| B-silanol/muramyl dipeptide ... | 32 |
| C-silanol seul ................ | 59 |
| D-sérine seule ................ | 96 |
| E-muramyl-dipeptide seul ...... | 43 |

On voit que le complexe silanol-sérine (A), et dans une moindre mesure le silanol seul (C), se comportent donc comme des activateurs des macrophages (et autres cellules du système réticulo-endothélial). Cette activation est, pour le silanol-sérine, du même ordre de grandeur que l'activation obtenue avec le muramyl-dipeptide seul (E) qui est un activateur bien connu des macrophages. Le complexe du silanol avec le muramyl-dipeptide (B) a des effets encore plus puissants.

Les solutions des complexes selon les exemples 2 et 4 sont utilisables en ampoules scellées à des fins thérapeutiques en vue de stimuler l'activité des macrophages, du système réticulo-endothélial et de stimuler ainsi les défenses immunitaires de l'organisme.

EXEMPLE 11

Essais du complexe silanol-caséine en vue de ses applications en tant qu'agent de régénération du tissu conjonctif dermique et sous-dermique

La solution préparée selon l'exemple 5, du complexe silanol caséine est utilisée en application cutanée. Cette solution constitue en particulier un produit efficace contre les rides qu'elle supprime ou atténue en stimulant la croissance du tissu conjonctif et la production de fibres élastiques et collagènes. Ce phénomène doit être rapproché de l'action du silanol-disérine sur la croissance et la multiplication de fibroblastes en culture (exemple 9) et de celle du silanol-sérine sur l'activation des macrophages (exemple 10), puisqu'il est connu

que les macrophages activés produisent eux-mêmes diverses hormones locales, stimulant la croissance des fibroblastes et la production des fibres élastiques et collagènes.

EXEMPLE 12

Essais du complexe silanol-hydroxyproline en vue de ses applications en tant qu'agent thérapeutique de l'ostéoporose

La solution préparée selon l'exemple 6, du complexe silanol-hydroxyproline a été injectée intrapéritonéalement à 15 rates âgées de 2 ans à raison de 0,2 ml soit 0,25 mg de Si par animal tous les 2 jours pendant 3 mois (lot A). Du silanol seul (lot B), de l'hydroxyproline seule (lot C) et du salin (lot D) ont été administrés de la même façon à 3 autres lots de 15 rates de même âge. Après trois mois de traitement, les animaux sont sacrifiés, un examen histologique est effectué sur la crête iliaque pour la mesure du volume trabéculaire osseux (en % de la surface totale) et le calcium total est déterminé sur le fémur de chaque animal.

RESULTATS

| Lot de rates | Produits | Variations (%) | |
|---|---|---|---|
| | | Volume trab. | Calcium |
| A | Silanol-hydroxyproline... | +24 | +15 |
| B | Silanol seul ........... | +18 | +12 |
| C | Hydroxyproline seule .... | + 3 | - 2 |

(variations exprimées en % des valeurs du lot témoin D).

Le silicium est connu pour jouer un rôle important dans les phénomènes de calcification osseuse ; d'autre part, l'hydroxyproline est un acide aminé nécessaire à la formation de la matrice organique de l'os. Les résultats obtenus montrent que le silicium administré sous forme de silanol augmente le processus de calcification osseuse des rates sénescentes, sujettes à l'ostéoporose. Le complexe silanol-hydroxyproline selon l'exemple 7, a des effets plus prononcés que le silanol seul, suggérant le rôle de l'hydroxyproline comme vecteur possible du silicium dans l'ostéoblaste, cellule responsable de la calcification osseuse. La solution à 1,25% obtenue suivant l'exemple 7 constitue un médicament contre l'ostéoporose.

En conclusion, les exemples 7 à 12 mettent en évidence des propriétés nouvelles du silanol complexé avec des protides; la plupart de ces propriétés nouvelles sont également manifestées mais à un moindre degré par le silanol seul. Ces nouvelles activités biologiques portent principalement sur la régulation de la croissance et de la multiplication des cellules humaines en culture (stimulation des cellules quiescentes, inhibition des cellules tumorales), sur la normalisation de la croissance des cellules tumorales, notamment agrégation des cellules en culture, et sur l'activation des macrophages (et autres cellules réticulo-endothéliales) et des ostéoblastes. Ces divers effets entraînent secondairement une immunostimulation généralisée, conséquence de la multiplication des lymphocytes et de l'activation des macrophages ; ils produisent une régénération du tissu conjonctif, conséquence de la multiplication des fibroblastes et de la production des fibres élastiques et collagènes, directement et/ou indirectement par l'activation des macrophages ; ils ont aussi une action favorable sur la calcification osseuse. Les solutions des exemples 1 à 7 et d'autres similaires, comme définies selon l'invention, constituent donc des médicaments immunostimulateurs, antitumoraux, régénérateurs et recalcifiants. Parmi les autres applications thérapeutiques possibles de ces produits, on peut citer l'utilisation à titre d'adjuvant dans ces vaccins, pour stimuler la production d'anticorps induite par ceux-ci. Les effets produits par l'immunostimulation s'exercent tant contre les bactéries que vis-à-vis des virus.

EXEMPLE 13

Essais du complexe silanol-sérine sur la prolifération bactérienne

La solution à 10 g de complexe par litre, préparée suivant l'Exemple 2, titrant 1 g Si/l, est diluée et ajoutée à une culture de Mycobacterium aurum de façon à titrer 10 mg Si par litre de culture.
Ni le silanol-sérine, ni le silanol seul, ni la sérine seule ne modifient la croissance de Mycobactérium aurum. On voit ainsi que les complexes silanol acides aminés, pas plus que le silanol seul, n'ont sur la multiplication

de bactéries les mêmes effets que ceux des Exemples 7 à 9 sur les cellules humaines.

## Revendications

1. Produit thérapeutique, comprenant un dérivé organique du silicium et un amino-acide, caractérisé en ce qu'il est constitué par une combinaison d'un silanol avec un amino mono-acide sans fonction phénol, ni thiol, libre ou à l'état d'un sel pharmaceutiquement acceptable, ou sous la forme de protide.

2. Produit suivant la revendication 1, constitué par une combinaison de silanol avec un protide, caractérisé en ce que celui-ci est un protide glycosylé.

3. Produit suivant la revendication 2, caractérisé en ce que le protide glycosylé est le muramyl dipeptide.

4. Produit suivant la revendication 1, 2 ou 3, caractérisé en ce que l'amino-acide est choisi parmi ceux qui existent dans la nature sous une forme libre ou combinée.

5. Produit suivant une des revendications précédentes, caractérisé en ce que l'amino-acide qu'il contient est lévogyre.

6. Produit suivant une des revendications précédentes, caractérisé en ce qu'il est constitué par un complexe qui contient 0,25 à 2 atomes Si du dérivé organique du silicium par groupe -COOH, $>$CHOH ou $-NH_2$ de l'acide aminé ou du protide.

7. Produit suivant une des revendications précédentes, caractérisé en ce que l'amino-acide est hydroxyle, notamment sérine, thréonine, homosérine, disérine, hydroxyproline ou hydroxylysine.

8. Produit suivant une des revendications 1 à 5, caractérisé en ce que l'amino-acide est la lysine, histidine, glycine, glutamine, asparagine, arginine ou ornithine.

9. Produit suivant une des revendications 1 à 8, dans lequel le silanol répond à la formule $R_xSi(OM)_{(4-x)}$, où R est un alkyle en $C_1$-$C_{18}$, un alcényle ayant jusqu'à 18 atomes C, ou un aryle en $C_6$ ou $C_{10}$ pouvant porter des substituants alkyliques en $C_1$ à $C_6$, x est un nombre de 0 à 3, et M désigne un atome de métal alcalin ou d'hydrogène.

10. Produit suivant la revendication 9, dans lequel R est un alkyle en $C_1$ à $C_4$ ou un alcényle ayant jusqu'à 4 atomes C.

11. Produit suivant une des revendications précédentes, caractérisé en ce qu'il est sous la forme d'une solution aqueuse, renfermant 0,1 à 1,4 g Si par litre.

12. Agent stimulateur de la croissance et de la multiplication des cellules eucaryotes normales, mais non des organismes bactériens, caractérisé en ce qu'il contient un produit suivant une des revendications 1 à 11.

13. Agent régulateur du métabolisme cellulaire inhibant la prolifération anarchique et normalisant la croissance des cellules tumorales, caractérisé en ce qu'il contient un produit suivant une des revendications 1 à 11.

14. Agent activateur du système immunitaire et en particulier des cellules du système réticulo-endothélial, caractérisé en ce qu'il contient un produit suivant une des revendications 1 à 11.

15. Agent régénérateur du tissu conjonctif pour action stimulatrice ou régulatrice des métabolismes cellulaires des éléments constitutifs des tissus conjonctifs d'une façon directe ou indirecte tels des mélanocytes, adipocytes, ostéoblastes, fibroblastes ou macrophages, caractérisé en ce qu'il contient un produit suivant une des revendications 1 à 11.

16. Agent favorisant la calcification osseuse en conséquence de son action sur les ostéoblastes, caractérisé en ce qu'il contient un produit suivant une des revendications 1 à 11.

## Claims

1. Therapeutic product, comprising an organic silicon derivative and an amino acid, characterized in that it is formed of a combination of a silanol with a monoamino acid having no phenol function, nor thiol function, the acid being free or in the state of a pharmaceutically-acceptable salt, or in the form of a protide.

2. Product according to claim 1 formed by a combination of silanol with a protide, characterized in that the protide is glycosylated.

3. Product according to claim 2, characterized in that the glycosylated protide is muramyldipeptide.

4. Product according to claim 1, 2 or 3, characterized in that the amino acid is selected from those which occur naturally in free or combined form.

5. Product according to one of the preceding claims, characterized in that the amino acid which it contains is levogyre.

6. Product according to one of the preceding claims, characterized in that it is constituted by a complex which contains 0.25 to 2 atoms of Si of the organic silicon derivative per -COOH, = CHOH or $-NH_2$ group of the amino acid or protide.

7. Product according to one of the preceding claims, characterized in that the amino acid contains a hydoxyl group, being in particular serine, threonine, homoserine, diserine, hydroxyproline or hydroxylysine.

8. Product according to one of claims 1 to 5, characterized in that the amino acid is lysine, histidine, glycine, glutamine, tryptophan, asparagine, or ornithine.

9. Product according to one of claims 1 to 8, characterized in that the silanol has the following formule $R_xSi$ (OM) $_{(4-x)}$ where R is an alkyl having from 1 to 18 carbon atoms, alkenyl having up to 18 carbon atoms or aryl having 6 or 10 carbon atoms and which can carry alkyl substituents in $C_1$, to $C_6$, x a number from 0 to 3, M designates an alkali metal atom or hydrogen.

10. Product according to claim 9, wherein R is an alkyl having from 1 to 4 carbon atoms or an alkenyl having up to 4 carbon atoms.

11. Product according to one of the preceding claims characterized in that it is in the form of an aqueous solution containing 0.1 to 1.4 g of Si per liter.

12. Agent for stimulation of the growth and multiplication of normal eukaryotic cells, but not bacterial organisms, characterized in that it contains a product according to one of claims 1 to 11.

13. Regulating agent for cellular metabolism inhibiting the anarchic proliferation and normalising the growth of tumoral cells, characterized in that it contains a product according to one of claims 1 to 11.

14. Activating agent for the imuno-system, particularly cells of the reticulo-endothelial system, characterized in that it contains a product according to one of claims 1 to 11.

15. Regenerating agent for conjunctive tissue for stimulatory or regulatory action, in a direct or indirect manner, of cell metabolism of elements constituting the conjunctive tissues such as melanocytes, adipocytes, osteoblasts, fibroblasts or macrophages, characterized in that it contains a product according to one of claims 1 to 11.

16. Agent for promoting bone calcification in consequence of its action on osteoblats, characterized in that it contains a product according to one of claims 1 to 11.

## Patentansprüche

1. Therapeutisches Produkt, welches ein organisches Siliciumderivat und eine Aminosäure umfaßt, dadurch gekennzeichnet, daß es von einer Kombination eines Silanols mit einer Monoaminosäure ohne Phenol- oder Thiolfunktion, frei oder im Zustand eines pharmazeutisch annehmbaren Salzes oder in Form eines Proteids, gebildet ist.

2. Produkt nach Anspruch 1, gebildet von einer Kombination von Silanol mit einem Proteid, dadurch gekennzeichnet, daß dieses ein glykosyliertes Proteid ist.

3. Produkt nach Anspruch 2, dadurch gekennzeichnet, daß das glykosylierte Proteid Muramyldipeptid ist.

4. Produkt nach Anspruch 1, 2 oder 3, dadurch gekennzeichnet, daß die Aminosäure aus denen ausgewählt ist, die in der Natur in freier Form oder kombiniert vorliegen.

5. Produkt nach einem der vorstehenden Ansprüche, dadurch gekennzeichnet, daß die Aminosäure, die es enthält, linksdrehend ist.

6. Produkt nach einem der vorstehenden Ansprüche, dadurch gekennzeichnet, daß es von einem Komplex gebildet ist, der 0,25 bis 2 Si-Atome des organischen Siliciumdericats pro -COOH-, $>$ CHOH- oder $-NH_2$-Gruppe der Aminosäure oder des Proteids enthält.

7. Produkt nach einem der vorstehenden Ansprüche, dadurch gekennzeighnet, daß die Aminosäure hydroxyliert ist, insbesondere Serin, Threonin, Homoserin, Diserin, Hydroxyprolin oder Hydroxylysin.

8. Produkt nach einem der Ansprüche 1 bis 5, dadurch gekennzeichnet, daß die Amirosäure Lysin, Histidin, Glycin, Glutamin, Asparagin, Arginir oder Ornithin ist.

9. Produkt nach einem der Ansprüche 1 bis 8, in welchem das Silanol der Formel $R_xSi(OM)_{(4-x)}$ entspricht, worin R ein $C_1$-$C_{18}$-Alkyl ist, ein Alkenyl mit bis zu 18 C-Atomen, ein $C_6$- oder $C_{10}$-Aryl, das $C_1$-$C_6$-Alkylsubstituenten tragen kann, ist, x eine ganze Zahl von 0 bis 3 ist und M ein Alkalimetallatom oder Wasserstoff bezeichnet.

10. Produkt nach Anspruch 9, worin R ein $C_1$-$C_4$-Alkyl oder ein Alkenyl mit bis zu 4 C-Atomen ist.

11. Produkt nach einem der vorstehenden Ansprüche, dadurch gekennzeichnet, daß es in Form einer wässrigen Lösung vorliegt, die 0,1 bis 1,4 g Si pro Liter enthält.

12. Stimulationsmittel für das Wachstum und die Vermehrung von normalen eukariontischen Zellen, jedoch

nicht von bakteriellen Organismen, dadurch gekennzeichnet, daß es ein Produkt nach einem der Ansprüche 1 bis 11 enthält.

13. Regulationsmittel für den zellulären Metabolismus, das die anarchische Vermehrung inhibiert und das Wachstum von Tumorzellen normalisiert, dadurch gekennzeichnet, daß es ein Produkt nach einem der Ansprüche 1 bis 11 enthält.

14. Aktivirungsmittel für das Immunsystem mund insbesondere für Zellen des retikulo-endothelialen Systems, dadurch gekennzeichnet, daß es ein Produkt nach einem der Ansprüche 1 bis 11 enthält.

15. Regenerationsmittel für Bindegewebe für die, auf direkte oder indirekte Weise, stimulierende oder regulierende Beeinflussung zellulärer Metabolismen der Bauelemente der Bindegewebe, wie der Melanozyten, Adipozyten, Osteoblasten, Fibroblasten oder Makrophagen, dadurch gekennzeichnet, daß es ein Produkt nach einem der Ansprüche 1 bis 11 enthält.

16. Mittel, das als Folge seiner Wirkung auf die Osteoblasten die Kalkbildung im Knochen begünstigt, dadurch gekennzeichnet, daß es ein Produkt nach einem der Ansprüche 1 bis 11 enthält.